# EUROPEAN PATENT SPECIFICATION

(11) **EP 3 234 142 B1**
(45) Date of publication and mention of the grant of the patent: **29.08.2018**
(21) Application number: 15745259.0
(22) Date of filing: 02.07.2015
(51) Int. Cl.: C12N 15/115, C12Q 1/68, A61K 31/713, A61P 35/00

(54) **DNA APTAMER RECOGNISING HUMAN PCNA PROTEIN AND USE THEREOF**
MENSCHLICHES PCNA-PROTEIN ERKENNENDES DNA-APTAMER UND VERWENDUNG DAVON
APTAMÈRE ADN RECONNAISSANT LA PROTÉINE PCNA HUMAINE ET SON UTILISATION

(30) Priority: 16.12.2014 PL 41057214
(43) Date of publication of application: 25.10.2017
(73) Proprietor: Uniwersytet Jagiellonski, 31-007 Krakow (PL)
(72) Inventor: BARTNICKI, Filip, PL-30-868 Kraków (PL); KOWALSKA, Ewa, PL-31-422 Kraków (PL); STRZALKA, Wojciech, PL-30-373 Kraków (PL)
(74) Representative: BRANDPAT Patent and Trademark Attorneys Chlebicka Czyz Galazkiewicz Ziolkowski Professional Partnership
(86) International application number: PCT/PL2015/050026
(87) International publication number: WO 2016/099310

(56) References cited:
- WO-A1-2012/082069
- WO-A1-2014/185802
- E. KOWALSKA ET AL: "The impact of immobilized metal affinity chromatography (IMAC) resins on DNA aptamer selection", ANALYTICAL AND BIOANALYTICAL CHEMISTRY, vol. 406, no. 22, September 2014 (2014-09) , pages 5495-5499, XP055218783, DE ISSN: 1618-2642, DOI: 10.1007/s00216-014-7937-y

## Description

The subject of the invention is a unique nucleotide sequence of a DNA aptamer binding human PCNA protein and its application.
PCNA (Proliferating Cell Nuclear Antigen) - a nuclear antigen of a dividing cell is a key eukaryotic protein involved in DNA replication and repair processes as well as in the regulation of the cell cycle. This protein was discovered as an autoantigen reacting with antibodies present in the serum of patients suffering from systemic lupus erythematosus. Crystallographic analysis of PCNA revealed that three monomers of this protein joined together form a ring-shaped structure. During DNA replication, the RFC (Replication Factor C) complex loads PCNA on a double helix where it slides on the DNA serving as a platform coordinating the action of other proteins engaged in DNA synthesis.DNA aptamers are defined as single-stranded deoxyribonucleic acid molecules about 40-100 nucleotides long, which have an ability to specifically bind to a particular molecular target. Optimal matching of the aptamer to the specific molecular target is possible and depends on secondary and tertiary structures of single-stranded DNA molecule.
The aim of the invention is to provide a new method of identification of human PCNA protein, especially through the provision of a new molecule with high affinity for PCNA. Unexpectedly, it turned out that the technical aim described above can be realised using this invention.
The subject of the invention is the DNA aptamer showing affinity for human PCNA protein and containing a nucleotide sequence SEQ ID. No. 1: 
5'-CATGCTTCCCCAGGGAGATGCCTATGGTCCCCGCGTAGGTGGCAGCTCAAACCCGATTCGAGGAA
CATGCGTCGCAAAC-3'
 subsequently also referred to as anti-PCNA aptamer.
Certain variability of the DNA sequence according to the invention is acceptable, on condition that its affinity for PCNA is preserved, enabling selective binding of this protein. The scope of the invention particularly encompasses the variants of the above-mentioned sequence, which differ between themselves by at least one purine or pyrimidine base.
Another subject of the invention is application of the oligonucleotide described above to produce a molecule with affinity for PCNA or molecular targets containing PCNA, especially:
- to detect PCNA,
- to bind PCNA,
- to purify PCNA,
- to obtain molecular targets complexed with PCNA,
- to inhibit cell divisions in the selected cancer cell lines. According to the invention, in particular aptamer in the form of molecules with attached markers, ex. biotin, can be used to label PCNA.
These kinds of molecules can be used to, for example, analyse the interactions between proteins using molecular biology techniques known to the specialist, such as the "pull down" method.
In order to better explain the subject of the invention defined above, the following figures were added to this description:
Figure 1 shows binding between human PCNA protein and the developed aptamer. Lanes: M) protein marker, 1) His-PCNA protein used in the experiment, 2) the result of binding the His-PCNA protein to the anti-PCNA aptamer, 3) the result of binding the His-PCNA protein to the reference aptamer consisting of the respective amount of repetitions of GATC sequence. The samples were separated in 12% denaturing polyacrylamide gel and then stained using Coomassie Brilliant Blue R-250.
Figure 2 shows the purification process of human PCNA protein from the protein extract prepared from *E. coli* cells. Lanes: M) protein marker, 1) 40 µg of protein extract prepared from *E. coli* cells, 2) 40 µg of protein extract prepared from *E. coli* cells, containing human PCNA protein, 3) 4 µg of PCNA protein purified using chromatographic resin containing anti-PCNA aptamer. The samples were separated in 12% denaturing polyacrylamide gel and then stained using Coomassie Brilliant Blue R-250.
Figure 3 depicts detection of human recombinant PCNA protein containing His-tag by ELISA method using the anti-PCNA aptamer. Presented result is the mean from three independent experiments.
Figure 4 presents results from MTT test where cytotoxic impact of anti-PCNA aptamer on the cells from LNCaP prostate cancer cell line was tested.. Presented result is the mean from three independently repeated experiments.
Figure 5 displays the % of PCNA protein in complex with anti-PCNA aptamer in the function of concentration of this aptamer. Moreover, this description has been supplemented with examples of implementation given below. They should not be identified with a full scope of the invention defined above.

### Example 1. Obtaining a DNA aptamer with affinity for human PCNA.

As a result of research aimed at developing a ssDNA molecule showing affinity for PCNA, it has been established that a DNA aptamer of the following sequence: 
5'-CATGCTTCCCCAGGGAGATGCCTATGGTCCCCGCGTAGGTGGCAGCTCAAACCCGATTCGAGGAA
CATGCGTCGCAAAC-3'
(SEQ ID. No. 1)
 has strong affinity for this protein.
According to the invention, a DNA molecule can be obtained using any routine method of DNA synthesis known to the specialist.

The oligonucleotide molecule was obtained using solid phase chemical synthesis technique (Zlatev, I., Manoharan, M., Vasseur, J.-J. and Morvan, F. Solid-Phase Chemical Synthesis of 5'-Triphosphate DNA, RNA, and Chemically Modified Oligonucleotides. Current Protocols in Nucleic Acid Chemistry. 2012; 50:1.28.1-1.28.16.)
The obtained anti-PCNA aptamer molecule of the sequence defined above, 79 nucleotides long, synthesised in the amount of 576 micrograms, was verified using the HPLC method. According to the invention, the DNA molecule containing the sequence can be additionally modified, especially coupled to known dyes (ex. fluorescent dyes) or other molecules (ex. biotin).
For example, biotinylated anti-PCNA aptamer was obtained in the respective amount of 434 micrograms using the method of automated synthesis of 5'-biotinylated oligonucleotides. (Pon, R.T. A Long Chain Biotin Phosphoramidite Reagent for The Automated Synthesis of 5'-Biotinylated Oligonucleotides, Tetrahedron Lett.1991; 32: 1715-1718). The obtained product was verified using the HPLC method.

### Example 2. Binding of human PCNA protein to chromatographic resin containing the aptamer according to the invention.

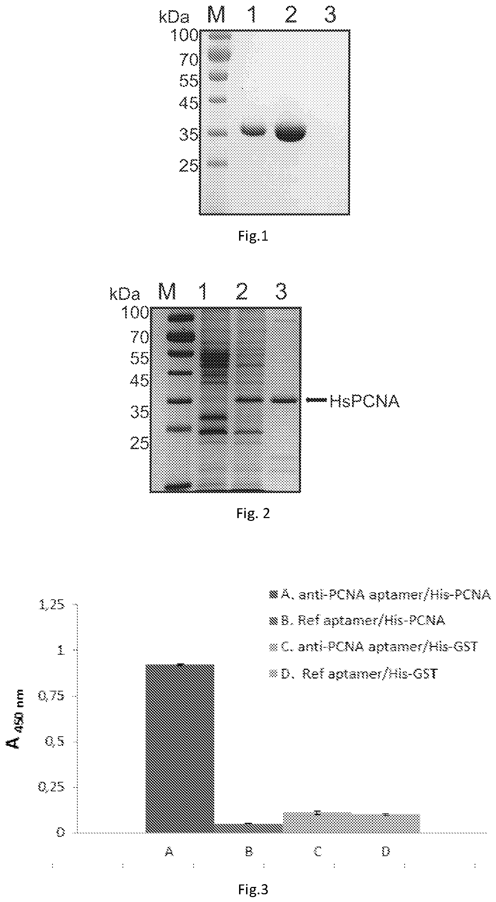

10 µl of 50% streptavidin-agarose was placed in a 1.5 ml eppendorf tube and washed with distilled water. It was then washed three times with the BW buffer (17.5 g/L NaCl; 50 mM phosphate buffer NaH₂PO₄/Na₂HPO₄; 0.1% (v/v) Tween 20; pH 7.5). The resin was then suspended in 300 µl of the BW buffer, and 20 µg of 5'-biotinylated anti-PCNA aptamer or a reference aptamer with the (ACTG)x20 sequence was added. The samples were incubated at room temperature for 1h. After the incubation, the resin was washed three times with the BW buffer followed by two washes with the AS buffer (137mM NaCl; 12.7mM KCl; 10mM Na₂HPO₄; 2mM KH₂PO₄; 5mM MgCl₂; 0.1% (v/v) Tween 20; pH 7.4). After the last washing, the resin with the bound aptamer was suspended in 300 µl of the AS buffer, and the human recombinant His-PCNA protein was added, to achieve a final concentration of 1 mg/ml. The resin was incubated at room temperature for 1h. After the incubation it was washed four times with the AS buffer. Then, the GLB buffer (50 mM Tris-HCl, 2% SDS (w/v), 2% bromophenol blue (w/v), 10% glycerol (v/v), 200 mM β-mercaptoethanol, pH 6.8) was added and they were incubated for 5 min at 95°C. The obtained sample was subjected to denaturing poliacrylamide gel electrophoresis (SDS-PAGE, Laemmli method) in 12% polyacrylamide gel. After the separation, the proteins were stained using Coomassie Brilliant Blue R-250 dye. The results are shown in Figure 1.

### Example 3. Purification of human PCNA protein from the E. coli cells total protein extract.

10 µl of 50% streptavidin-agarose resin was placed in a 1.5 ml eppendorf tube and washed with distilled water. It was then washed three times with the A buffer (50 mM Tris; 300 mM NaCl, 0.01% (v/v) Tween 20; pH 7.5). The resin was then suspended in 300µl of the A buffer and 20 µg of 5'-biotinylated anti-PCNA aptamer was added. The samples were incubated at room temperature for 1h. After the incubation, the resin was washed three times with the A buffer. After the last washing, the resin with the bound aptamer was suspended in 300 µl of the A buffer, and the protein extract prepared from *E. coli* cells, containing human recombinant PCNA protein was added, to achieve a final total protein concentration of 16 mg/ml. The resin was incubated at 4°C for 1h. After the incubation, it was washed four times with the A buffer. Then, the PCNA protein was eluted from the resin using an elution buffer (50mM Tris, 500mM NaCl, 300mM guanidine hydrochloride, pH 7.5). The preparation composed of the eluted protein was mixed with the GLB buffer (50 mM Tris, 2% SDS (w/v), 2% bromophenol blue (w/v), 10% glycerol (v/v), 200 mM β-mercaptoethanol, pH 6.8) and incubated for 5 min at 95°C. The obtained sample was subjected to denaturing poliacrylamide gel electrophoresis (SDS-PAGE, Laemmli method) in 12% polyacrylamide gel. After separation, the proteins were stained using Coomassie Brilliant Blue R-250 dye. The results are shown in Figure 2.

### Example 4. Analysis of human PCNA protein using ELISA method.

A 96-well ELISA test plate with immobilised nickel ions was coated with His-PCNA or His-GST protein (0.5 µg of protein/well) at 4°C for 16h. The plate was then washed three times with a PBST buffer (1x PBS containing 0.5% (v/v) Tween 20). In the next step, the plate was blocked with 1x PBS containing 1% (w/v) bovine albumin at room temperature for 2h. The plate was then washed three times with the PBST buffer and the biotinylated anti-PCNA aptamer or the reference aptamer (of sequence (ACTG)x20) dissolved in the AS buffer (137mM NaCl; 12.3mM KCl; 10mM Na₂HPO₄; 2mM KH₂PO₄; 5mM MgCl₂; pH 7.4; 0.1% (v/v) Tween 20) was added to achieve final aptamer concentration of 0.1 mg/ml. The incubation was carried out at room temperature for one hour. The plate was then washed three times with the PBST buffer, horseradish peroxidase coupled to streptavidin (dilution 1:200 in the PBS buffer) was added to the wells and the incubation was carried out for 40 minutes at room temperature. Afterwards, the plate was washed three times with the PBST buffer and a substrate for horseradish peroxidase was added to the wells. When a blue colour was observed, the reaction was stopped using 1M H₂SO₄ and the absorbance was measured at the wavelength of 450 nm. The obtained results are shown in Figure 3.

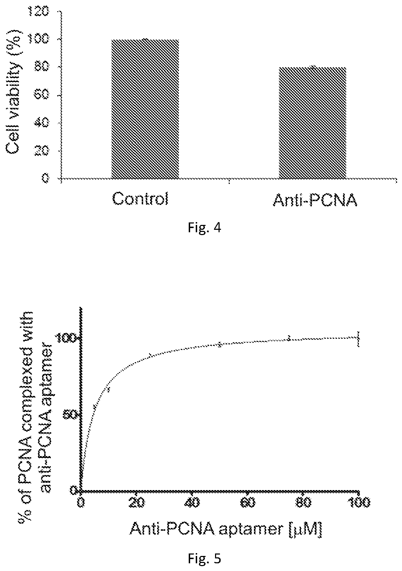

### Example 5. Inhibition of cell divisions in the selected cancer cells lines (Fig. 4).

Cells from LNCaP epithelial cancer cell line (ATCC® CRL-1740™) were inoculated on a 96-well plate in the amount of 1000 cells/well. After culturing for 16h in the incubator (37°C, 5% CO₂), the anti-PCNA aptamer or the reference aptamer (of sequence (ACTG)20x) (dissolved in fresh culture medium RPMI-1640 containing 10% bovine serum) was added to the cells, to achieve final concentration of 33 µM. After 24 and 48h of culturing, the medium was replaced with a fresh one, containing the analysed aptamers. After 72h of cell growth in the presence of the aptamers, an MTT test showing the activity of energetic changes in mitochondria was performed to reflect the relative cell viability. In order to do this, after incubation with the aptamers, the cells were washed with the PBS buffer and 100 µl of the fresh culture medium containing MTT reagent (Thiazolyl Blue Tetrazolium Bromide, yellow colour) of the final concentration of 0.5 mg/ml was added. After 4h of incubation, crystals of insoluble formazan (dark blue colour), formed as a result of the reduction of MTT reagent, were dissolved using isopropanol with 0.04M HCl. Formazan level, which is directly proportional to the number of live cells, was assessed by colorimetric method using absorbance measurement at 540 nm wavelength.

### Example 6. Determination of the human PCNA protein/anti-PCNA ssDNA aptamer complex dissociation constant (Fig. 5).

40 µl of 50% streptavidin-agarose resin was placed in 1.5 ml eppendorf tubes. The resin was washed once with distilled water, then three times with the AS buffer (137 mM NaCl; 12.7 mM KCl; 10 mM Na₂HPO₄; 2 mM KH₂PO₄; 5mM MgCl₂; 0.1% (v/v) Tween 20; pH 7.4;). The resin was then suspended in 100 µl of the AS buffer containing the proper amount of 5'-biotinylated variant of anti-PCNA aptamer (of sequence: 5'-CATGCTTCCCCAGGGAGATG CCTATGGTCCCCGCGTAGGTGGCAGCTCA-3') so that during further incubation with the PCNA protein, the aptamer concentration amounted to: 0; 0.5; 1.0; 2.5; 5.0; 10.0; 25.0; 50.0; 75.0 or 100.0 µM, respectively. The samples were incubated at room temperature for 1h, and then washed five times with the AS buffer. The resin was then suspended in the AS buffer containing 100 nM human His-PCNA protein and incubated at room temperature for 1h. After that, the supernatants were transferred into new eppendorf tubes and the GLB buffer (50 mM Tris-HCl, 2% SDS (w/v), 2% bromophenol blue (w/v), 10% glycerol (v/v), 200 mM β-mercaptoethanol, pH 6.8) was added, and the samples were incubated for 5 min at 95°C. The obtained preparations were subjected to denaturing poliacrylamide gel electrophoresis (SDS-PAGE, Laemmli method) in 12% polyacrylamide gel. After separation, the proteins were stained using Coomassie Brilliant Blue R-250 dye. Based on the densitometric measurements, the percent of PCNA protein in complex with the studied aptamer was determined in each of the studied samples. The value of the dissociation constant for the studied complex was calculated using GraphPad Prism software, applying the model with one specific ligand-binding site. The value of the dissociation constant amounted to 5.08 µM.

## Claims

1. DNA aptamer containing a nucleotide sequence presented as SEQ ID. No. 1, showing the affinity for human PCNA protein.

2. Use of the oligonucleotide containing the sequence according to claim 1 in the production of molecules with affinity for PCNA and molecular targets containing PCNA, especially:
- to detect PCNA,
- to bind PCNA,
- to purify PCNA,
- to obtain molecular targets complexed with PCNA,
- to inhibit cell division in the selected cancer cell lines.

## Patentansprüche

1. DNA-Aptamer, umfassend eine Nukleotidsequenz dargestellt als SEQ ID. Nr. 1, das Affinität für das Humanprotein PCNA zeigt.

2. Verwendung des Oligonukleotids, welches die Sequenz nach Anspruch 1 umfasst, bei der Produktion von Molekülen mit Affinität für PCNA und PCNA-enthaltende Zielmoleküle, insbesondere:
- um PCNA zu detektieren,
- um PCNA zu binden,
- um PCNA aufzureinigen,
- um mit PCNA komplexierte Zielmoleküle zu erhalten,
- um Zellteilung in den ausgewählten Krebszelllinien zu hemmen.

## Revendications

1. Aptamère d'ADN contenant une séquence nucléotidique présentée sous la forme SEQ ID. No. 1, montrant l'affinité pour la protéine PCNA humaine.

2. Utilisation de l'oligonucléotide contenant la séquence selon la revendication 1 dans la production de molécules ayant une affinité pour PCNA et de cibles moléculaires contenant PCNA, en particulier :
- pour détecter PCNA,
- pour se lier à PCNA,
- pour purifier PCNA,
- pour obtenir des cibles moléculaires complexées avec PCNA,
- pour inhiber une division cellulaire dans les lignées cellulaires cancéreuses choisies.
